# EUROPÄISCHE PATENTSCHRIFT

(11) **EP 0 486 840 B1**
(45) Veröffentlichungstag und Bekanntmachung des Hinweises auf die Patenterteilung: **14.06.1995**
(21) Anmeldenummer: 91118263.2
(22) Anmeldetag: 25.10.1991
(51) Int. Cl.: A61B 5/14

(54) **Blutentnahmevorrichtung**
Blood sampling device
Dispositif de grélèvement sanguin

(30) Priorität: 17.11.1990 DE 4036673
(43) Veröffentlichungstag der Anmeldung: 27.05.1992
(73) Patentinhaber: Walter Sarstedt Geräte und Verbrauchsmaterial für Medizin und Wissenschaft, D-51588 Nümbrecht (DE)
(72) Erfinder: Sarstedt, Walter, W-5223 Nümbrecht-Rommelsdorf (DE); Färber, Horst, W-5223 Nümbrecht (DE)

(56) Entgegenhaltungen:
- EP-A- 0 058 440
- DE-A- 1 566 094
- DE-A- 3 932 112

## Beschreibung

Die Erfindung betrifft eine Blutentnahmevorrichtung nach dem Oberbegriff des Patentanspruchs .

Es ist bereits eine Blutentnahmevorrichtung dieser Art vorgeschlagen worden (DE-A-39 32 112), bei der nach dem Herausziehen der Kanüle als der Vene im Innenrohr befindliches Blut aus dem Innenrohr in das Probenröhrchen hineinfließen kann, was durch einen beim Herausziehen des Innenrohrs im unteren Teil des Probenröhrchens erzeugten Unterdruck unterstützt werden kann.

Die Erfindung betrifft eine Verbesserung der gattungsgemäßen Blutentnahmevorrichtung dahingehend, daß eine axiale Relativverschiebung zwischen Innenrohr und Probenröhrchen dann, wenn sie unerwünscht ist, d.h. insbesondere während der Blutentnahme sicher vermieden wird, und daß nach der Blutentnahme im Innenrohr befindliches Blut vollständig aus diesem in das Probenröhrchen überführt wird, wenn das Innenrohr aus dem Probenröhrchen herausgezogen wird.

Zur Lösung dieser Aufgabe sind die Merkmale des kennzeichnenden Teils des Patentanspruchs vorgesehen.

Auf diese Weise ist gewährleistet, daß das Innenrohr bei der Blutentnahme fest mit dem Probenröhrchen verbunden werden kann und vorne durch die selbstschließende Membran dicht verschlossen ist, so daß während der Blutabnahme eine unerwünschte Relativverschiebung zwischen Innenrohr und Probenröhrchen nicht möglich ist .

Erst wenn nach der Blutentnahme die Axialsperrmittel gelöst werden und die Kappe axial abgenommen wird, kann Luft in das vordere Ende des Innenrohres eintreten, so daß bei dem aufgrund der Schleppverbindung erfolgenden Herausziehen des Innenrohres aus dem Probenröhrchen das im Innenrohr enthaltene Blut in das Probenröhrchen übertritt.

Die Erfindung wird im folgenden beispielsweise anhand der Zeichnung beschrieben; in dieser zeigt:
- Fig. 1: eine teilweise geschnittene Seitenansicht einer erfindungsgemäßen Blutentnahmevorrichtung in der Blutabnahmeposition und
- Fig. 2: einen Ausschnitt aus dem vorderen Teil der erfindungsgemäßen Blutentnahmevorrichtung nach Fig. 1 während des Abnehmens der Dichtkappe vom Probenröhrchen.

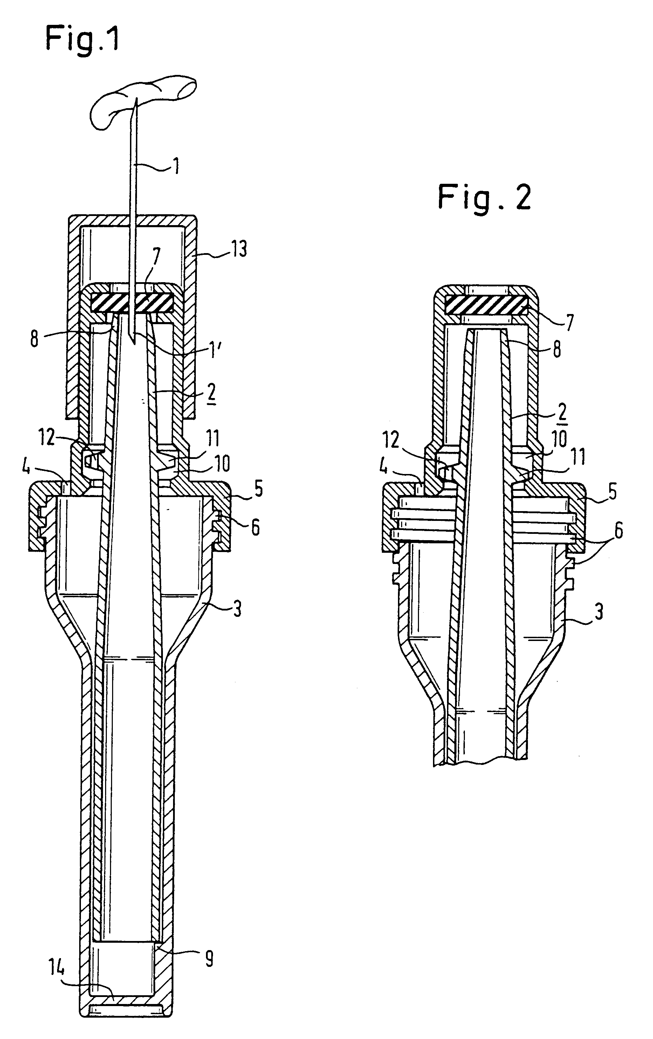

Nach Fig. 1 ist in einen sich oben erweiternden Probenröhrchen 3, das an seinem hinteren Ende 14 hermetisch abgeschlossen ist, ein Innenrohr 2 derart angeordnet, daß zwischen der Außenwand des Innenrohres 2 und der Innenwand des Probenröhrchens 3 noch ein Luftdurchlaß verbleibt. Das hintere Ende des Innenrohres 2 reicht in der in Fig. 1 wiedergegebenen Blutabnahmeposition bis nahe an das hintere Ende des Probenröhrchens 3 und stützt sich dort an einem an der Innenwand des Probenröhrchens 3 vorgesehenen Absatz 9 axial ab, der einen geringen Abstand vom Ende 14 aufweist.

Das vordere Ende des Probenröhrchens 3 ist durch eine Verschlußkappe 5 verschlossen, die mittels eines Gewindes 6 auf das vordere Ende des Probenröhrchens 3 aufgeschraubt ist. In der Verschlußkappe 5 ist eine Öffnung 4 vorgesehen, die den Innenraum des Probenröhrchens 3 am vorderen Ende entlüftet.

Im vorderen Teil der Verschlußkappe 5 ist eine durchstechbare und selbstschließende Membran 7 angeordnet, welche in der Blutentnahmeposition nach Fig. 1 das vordere Ende 8 des Innenrohres 2 dicht verschließt.

Eine ringförmige Aussparung 10 in der Innenwand der Verschlußkappe 5 nimmt einen am Innenrohr 2 vorgesehenen Ringflansch 11 mit einer Lüftungsöffnung 12 auf.

Auf den vorderen Teil der Verschlußkappe 5 ist eine eine beidseitig angeschärfte Kanüle 1 enthaltende Führungshülse 13 aufgeschoben, deren vorderes Ende in einer nur schematisch angedeuteten Vene steckt, während das hintere Ende 1' die Membran 7 durchstochen hat und in das Innere des Innenrohrs 2 reicht.

Die Funktion der beschriebenen Blutentnahmevorrichtung ist wie folgt:
In der Blutentnahmeposition nach Fig. 1 fließt Blut durch die Kanüle 1 in das Innere des Innenrohrs 2 . Sobald der gewünschte Füllungsgrad erreicht ist, beendet die Bedienungsperson die Blutabnahme, indem das Probenröhrchen 3 mit der mit ihr fest verbundenen Verschlußkappe 5 aus der Führungshülse 13 herausgezogen wird, wobei das hintere Ende 1' der Kanüle 1 nach vorn aus der Membran 7 heraustritt, welche sich dabei selbst schließt, so daß anschließend das vordere Ende 8 des Innenrohres 2 dicht verschlossen ist.

Anschließend wird dann die Verschlußkappe 5 vom Probenröhrchen 3 abgeschraubt und axial abgenommen. Hierbei hebt wegen des zunächst zwischen den Ringflansch 11 und der hinteren Begrenzung der Aussparung 10 vorhandenen Spiels (Fig. 1) die Membran 7 vom vorderen Ende 8 des Innenrohrs 2 ab, während anschließend nach Überwindung dieses Spiels aufgrund der nunmehr vorhandenen Schleppverbindung das Innenrohr 2 mitgenommen wird.

Hierbei tritt das im Innenrohr 2 befindliche Blut wegen der vorhandenen Entlüftung des vorderen Endes 8 des Innenrohres 2 über die Entlüftungsbohrungen 4, 12 unten aus dem Innenrohr 2 in das Probenröhrchen 3 über. Dadurch befindet sich das abgenommene Blut nach dem vollständigen Herausziehen des Innenrohres 2 vollständig im Probenröhrchen 3.

## Patentansprüche

1. Blutentnahmevorrichtung mit einer mit ihrem vorderem Ende in die Vene eines Patienten einstechbaren Kanüle (1), deren hinteres Ende (1') in ein Innenrohr (2) mündet, welches sich im Innern eines Probenröhrchens (3) befindet, das an seinem hinteren Ende hermetisch und an seinem vorderen Ende bis auf eine Entlüftung (4) geschlossen ist,
dadurch **gekennzeichnet,**
daß das Innenrohr (2) in der Blutabnahmeposition durch eine Kappe (5) mit lösbaren Axialsperrmitteln (6) vorzugsweise durch eine Schraubkappe gegen Herausziehen aus dem Probenröhrchen (3) gesichert ist, wobei in dem vorderen Teil der Kappe (5) eine durchstechbare und selbstschließende Membrane (7) angebracht ist, die in der Blutentnahmeposition das Innenrohr (2) an seinem vorderen Ende (8) dicht verschließt und gleichzeitig das Innenrohr (2) spielfrei gegen einen Anschlag im Probenröhrchen (3) drückt, vorzugsweise durch einen Absatz (9) am unteren Ende des Probenröhrchens (3), und daß das Innenrohr (2) und die Verschlußkappe (5) so mit einer Schleppverbindung miteinander verbunden sind, daß beim axialen Abnehmen der Kappe (5) von dem Probenröhrchen (3) zunächst die Dichtung zwischen Membrane (7) und Innenrohr (2) aufgehoben wird und erst anschließend das Innenrohr mitgenommen wird, vorzugsweise durch eine Aussparung (10) an der Kappe (5) und einen Ringflansch (11) mit Lüftung (12) am Innenrohr (2).

## Claims

1. Blood removing device having a cannula (1) which can be inserted by its front end into the vein of a patient and the rear end (1') of which opens out in an inner tube (2) arranged inside a sample tube (3) which is sealed hermetically at its rear end and is closed at its front end except for a ventilation opening (4), characterised in that the inner tube (2), in the blood removal position, is secured against withdrawal from the sample tube (3) by a cap (5) having releasable axial locking means (6), preferably by a screw cap, there being fitted in the front portion of the cap (5) a penetrable and self-closing diaphragm (7) which, in the blood removal position, seals the inner tube (2) tightly at its front end (8) and at the same time presses the inner tube (2) without play against a stop in the sample tube (3), preferably by means of a shoulder (9) at the lower end of the sample tube (3), and in that the inner tube (2) and the closure cap (5) are connected to one another by means of a tow connection in such a manner that, when the cap (5) is removed axially from the sample tube (3), the seal between the diaphragm (7) and the inner tube (2) is first suspended and only then is the inner tube entrained, preferably through a recess (10) in the cap (5) and an annular flange (11) having a ventilation opening (12) on the inner tube (2).

## Revendications

1. Dispositif de prélèvement de sang avec une canule (1) pouvant s'introduire avec son extrémité avant dans la veine d'un patient, dont l'extrémité arrière (1') débouche dans un tube intérieur (2), lequel se trouve à l'intérieur d'une éprouvette (3) qui à son extrémité arrière est fermée hermétiquement et à son extrémité avant est fermée à l'exception d'une aération (4), caractérisé en ce que le tube intérieur (2) en position de prélèvement de sang est protégé, de manière à pouvoir être extrait de l'éprouvette (3), par un capuchon (5) avec moyens de blocage axiaux (6) amovibles, de préférence par un capuchon à vis, dans la partie avant du capuchon (5) étant placée une membrane (7) à percer et se fermant d'elle-même, qui en position de prélèvement de sang ferme hermétiquement le tube intérieur (2) à son extrémité avant (8) et presse en même temps le tube intérieur (2), sans jeu, contre une butée à l'intérieur de l'éprouvette (3), de préférence par un épaulement (9) à l'extrémité avant de l'éprouvette (3), et en ce que le tube intérieur (2) et le capuchon de fermeture (5) sont reliés entre eux par une liaison d'entraînement telle que lorsque le capuchon (5) est enlevé de l'éprouvette (3), l'étanchéité entre la membrane (7) et le tube intérieur (2) est d'abord supprimée puis le tube intérieur est enlevé aussi, de préférence à travers une découpe (10) du capuchon (5) et une bride annulaire (11) avec aération (12) sur le tube intérieur (2).
